(19) Europäisches Patentamt
European Patent Office
Office européen des brevets

(11) **EP 3 393 426 B1**

(12) **EUROPEAN PATENT SPECIFICATION**

(45) Date of publication and mention
of the grant of the patent:
**19.02.2020 Bulletin 2020/08**

(21) Application number: **16805789.1**

(22) Date of filing: **01.12.2016**

(51) Int Cl.:
*A61K 8/25* *(2006.01)*    *A61K 8/26* *(2006.01)*
*A61Q 11/00* *(2006.01)*   *A61K 8/02* *(2006.01)*
*A61K 8/21* *(2006.01)*    *A61K 33/06* *(2006.01)*
*A61K 33/10* *(2006.01)*   *A61K 33/12* *(2006.01)*
*A61K 33/16* *(2006.01)*

(86) International application number:
**PCT/EP2016/079474**

(87) International publication number:
**WO 2017/108368 (29.06.2017 Gazette 2017/26)**

(54) **TOOTHPASTE COMPOSITION**

ZAHNPASTAZUSAMMENSETZUNG

COMPOSITION DE DENTIFRICE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.12.2015 EP 15201945**

(43) Date of publication of application:
**31.10.2018 Bulletin 2018/44**

(73) Proprietors:
• **Unilever NV**
**3013 AL Rotterdam (NL)**
Designated Contracting States:
**AL AT BE BG CH CZ DE DK EE ES FI FR GR HR
HU IS IT LI LT LU LV MC MK NL NO PL PT RO RS
SE SI SK SM TR**
• **Unilever PLC**
**London, Greater London EC4Y 0DY (GB)**
Designated Contracting States:
**CY GB IE MT**

(72) Inventors:
• **CHANDRASEKARAN, Sembian**
**Mumbai 400 099 (IN)**

• **IYER, Meenakshi**
**Mumbai 400 099 (IN)**
• **TRIVEDI, Neha**
**Mumbai 400 099 (IN)**

(74) Representative: **Tansley, Sally Elizabeth**
**Unilever N.V.**
**Unilever Patent Group**
**Olivier van Noortlaan 120**
**3133 AT Vlaardingen (NL)**

(56) References cited:
**EP-A2- 0 102 695        WO-A1-81/02670
WO-A1-2014/102032     WO-A2-2013/007571
CN-A- 104 334 150      JP-A- 2001 122 749
US-A- 4 064 231         US-A- 4 122 163**

• **DATABASE GNPD [Online] MINTEL; June 2015
(2015-06), Laboratoire Gravier: "Organic Lemon
Toothpaste", XP002754966, Database accession
no. 3216891**

EP 3 393 426 B1

**Description**

**Field of the invention**

The present invention relates to a toothpaste composition, it particularly relates to a toothpaste composition having fluoride source for enhancing remineralisation of tooth.

**Background of the invention**

Dental caries or cavities are common oral care problems, more commonly known as tooth decay. Dental caries is caused by demineralisation of tooth enamel.

Bacteria in the buccal cavity breaks down food, and in the process produces acid, the acid erodes tooth enamel which eventually results in dental caries. Calcium and phosphate compound which makes up the tooth enamel is a modified form of hydroxyapatite, which is susceptible to attacks by acid. To a certain extent saliva prevents erosion of tooth enamel by neutralizing these acids and the minerals in saliva deposit on the teeth to re-mineralize the enamel. As long as the rate of demineralization and the rate of re-mineralisation remain in balance, teeth remain strong and healthy. Imbalance in this process results in the formation of dental caries which occurs when more minerals are lost from teeth than can be replaced.

Fluoride is a known anti-caries agent. Fluoride helps in slowing down the demineralisation by interacting with hydroxyapatite to form a stronger compound that is less-susceptible to acid attack. Fluoride ions partially fluoridate hydroxyapatite and simultaneously repair the lattice irregularities and improves remineralisation process. The effectiveness of fluoride action depends upon the amount of fluoride ion which is available for deposition on the enamel. It is, therefore desirable to formulate toothpaste compositions which provide maximum fluoride ion availability in brushing solutions formed using the toothpaste composition.

Toothpaste with fluoride source are known in the art.

US4455293 B (Harvey et al., 1984) discloses a stable dentifrice formulation having a polishing agent comprising alkali metal aluminosilicate and a stabilising amount of mono-fluorophosphate ion.

EP0102695 A2 (Advanced Research & Technology Institute, 1985) discloses that compositions containing calcined kaolins have better rheological properties. Compositions disclosed in this publication contain calcined kaolin, talc and titanium dioxide as primary ingredients. The compositions also contain a source of fluoride.

US4064231 A (Kao Corp, 1977) discloses a dentifrice composition comprising a water-soluble fluoride and 0.3 to 13 wt% montmorillonite or hectorite having a specific composition leads to reduction of the water-soluble fluoride concentration in the dentifrice with the passage of time.

US4122163 A (Advanced Research & Technology Institute, 1978) discloses new and more effective dentifrice preparations containing calcined kaolin (a 1:1 clay) for abrasion and a source of fluoride. An example formulation contains Veegum® which is a 2:1 clay and trisodium citrate as the abrasive.

Incorporating a source of fluoride in a toothpaste composition is challenging. Primary limitation towards delivering high levels of fluoride is owing to the tendency of fluoride ions to be deactivated and rendered unavailable by other ingredients in the toothpaste composition, particularly the abrasive component. While known abrasives are compatible with a source of fluoride to varying degrees, there is a wide variation in the compatibility. Calcium-containing abrasives are not particularly compatible. While the non-calcium-containing abrasives are somewhat more compatible, but such abrasives are inferior in their ability to polish enamel.

It is therefore desired that the performance of the fluoride source in a toothpaste composition is improved and which delivers maximum benefit to tooth without compromising on the other benefits derivable from a toothpaste composition.

Accordingly, a first object of the present invention is to provide a toothpaste composition in which the incorporated fluoride gives higher order remineralisation. Yet another object of the present invention is to provide a toothpaste composition in which the incorporated fluoride is in a readily available form.

**Summary of the invention**

It is surprisingly found by the present inventors that a toothpaste composition having a source of fluoride provides for improved remineralisation of the tooth when the composition includes an abrasive silica or a calcium based abrasive, a 1:1 layered silicate clay and a 2:1 layered silicate clay.

The resulting toothpaste composition shows not only an increased fluoride availability when the product is first formulated, but also shows a significant resistance to degradation of fluoride availability during storage.

"Toothpaste" for the purposes of the present invention means a paste or gel composition for use with a toothbrush. Especially preferred are toothpaste composition suitable for cleaning tooth by brushing for about two minutes.

As used herein the term 1:1 layered silicate clay refers to a two-layer type clay in which sheet structures are composed of units of one layer of silica tetrahedron and one layer of alumina octahedron.

As used herein the term 2:1 layered silicate clay refers to a three-layer type clay in which sheet structures are composed of two layers of silica tetrahedrons and one central octahedron layer which is preferably a dioctahedral or a trioctahedral layer.

According to a first aspect disclosed is a toothpaste composition having:

(i) 2 to 70wt% abrasive;
(ii) a fluoride source;
(iii) 1:1 layered silicate clay; and,
(iv) 2:1 layered silicate clay,

wherein the abrasive is an abrasive silica or a calcium based abrasive.

According to a second aspect disclosed is a method of promoting remineralisation of teeth comprising the steps of:

(i) contacting teeth with the composition of the first aspect; and,
(ii) optionally, rinsing the oral cavity with a suitable solvent.

According to a third aspect is disclosed use of a 1:1 layered silicate clay together with a 2:1 layered silicate clay in a toothpaste composition comprising a fluoride source and an abrasive silica or a calcium based abrasive, for promoting remineralization of tooth.

According to a fourth aspect is disclosed use of a 1:1 layered silicate clay together with a 2:1 layered silicate clay in a toothpaste composition comprising a fluoride source and an abrasive silica or a calcium based abrasive, for decreasing demineralization of teeth.

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

All references to the term/expression wt% or % by weight, shall mean percentage by weight of the composition, except where indicated otherwise.

## Detailed description of the invention

According to a first aspect disclosed is a toothpaste composition having an abrasive, a source of fluoride, a 2:1 layered silicate clay and a 1:1 layered silicate clay.

### Abrasive

Disclosed toothpaste composition includes 2 to 70wt% abrasive wherein the abrasive is an abrasive silica or a calcium based abrasive.

The abrasive used in accordance with the invention is a particulate abrasive material such as a silica, calcium carbonate, dicalcium phosphate, calcium pyrophosphate, hydroxyapatiteor mixtures thereof. Agglomerated particulate abrasive materials may also be used.

Transparent or translucent gels usually contain silica whereas opaque creams generally contain calcium based abrasives, especially chalk. The quantity of the abrasive needs some control because excess abrasive causes more abrasion. Further, uncontrolled amount of abrasive also adversely affects viscosity of the toothpaste composition.

The abrasive is preferably present from 10 to 70% by weight of the composition. Preferably the amount of abrasive is 20 wt%, more preferably 25 wt%, still more preferably 30 wt%, further preferably 40wt% and most preferably 45 wt% of the composition. The abrasive employed in the disclosed invention is further preferably calcium based. It is

3

preferred that the disclosed composition includes 40 wt% to 60 wt%, more preferably 45 wt% to 55 wt% of the calcium based abrasive.

A particularly preferred calcium based abrasive is calcium carbonate. Fine ground natural chalk (FGNC), a form of chalk, is highly preferred. FGNC may also be modified chemically or physically by coating during milling or after milling by heat treatment. Typical coating materials include magnesium stearate and oleate. The morphology of FGNC may also be modified during the milling process by using various milling techniques, for example, ball milling, air-classifier milling or spiral jet milling. FGNC can be used as the sole calcium based abrasive. However, FGNC can also be used with other calcium based abrasives to balance the abrasion. Usually the particle size of chalk is from 1 to 60 micrometers, and the preferred size range from 1 to 15 micrometers. Other preferred calcium based abrasives include dicalcium phosphate (DCP), calcium pyrophosphate and precipitated calcium carbonate (PCC). When a combination of calcium based abrasives is used, it is preferred that FGNC is 35 to 100%, more preferably 75 to 100% and especially from 95 to 100% of the whole of the calcium based abrasives. In such cases, the balance, most preferably, is PCC (precipitated calcium carbonate).

Other abrasives may optionally also be used along with the calcium based abrasive, depending upon the intended degree of abrasion. These include synthetic abrasive polishing agents such as magnesium carbonate, sodium metaphosphate, potassium metaphosphate, zirconium silicate, potassium metaphosphate, magnesium orthophosphate, magnesium orthophosphate, trimagnesium phosphate, aluminum silicate, zirconium silicate and perlite.

Source of fluoride

Disclosed toothpaste composition includes a source of fluoride.

Preferably the source of fluoride is a soluble fluoride salt that provides a source of fluoride ions. Any orally acceptable fluoride salt or combination of salts may be used. Examples include, but are not limited to stannous fluoride, sodium fluoride, potassium fluoride, sodium monofluorophosphate, sodium fluorosilicate, ammonium fluorosilicate, amine fluoride, ammonium fluoride, and combinations thereof. Preferably the fluoride source is a stannous fluoride, sodium fluoride, sodium monofluorophosphate and mixtures thereof. It is preferred that the fluoride source is sodium monofluorophosphate.

Depending on the application, the toothpaste composition in accordance with the present invention may include free fluoride ion in a range of 1,000 to 20,000 ppm by weight of the composition. Preferably free fluoride ion concentration in a toothpaste composition for general consumer use would typically range from 1000 to 15000 ppm by weight of the composition. Source of fluoride is preferably added to the composition of the disclosed invention at a level of about 0.01 wt% to 10 wt%, more preferably 0.03 to 5 wt%, still more preferably 0.1 to 2 wt% and most preferably from 0.15 to 1wt% of the composition. The weight of the source of fluoride which is typically a fluoride salt, to provide the appropriate levels of the free fluoride ion will vary depending on the weight of the counter ion in the salt.

1:1 layered silicate clay

Disclosed toothpaste composition includes a 1:1 layered silicate clay. Typically the 1:1 layered silicate clay are non-swelling clays.

1:1 layered silicate clay includes kaolinite-serpentine group of clay. Kaolinite-serpentine group of clay includes subgroups of kaolinites and serpentines minerals. Serpentines are trioctahedral sheet minerals which has a tetrahedral sheet and an octahedral sheet having magnesium with minor amounts of aluminium and the species within this subgroup are preferably chrysotile, lizardite and antigorite.

Preferably the 1:1 layered silicate clay is a kaolinites clay. Kaolinites have a dioctahedral sheet and the species included within the kaolinites subgroup are kaolinite, dickite, nacrite and halloysite clay minerals, but not limited thereto. Kaolinite is particularly preferred in the disclosed invention.

Kaolinite, commonly known as kaolin clay, is a naturally occurring mineral. Kaolinite may be a calcined kaolin, highly purified calcined kaolin, colloidal kaolin, or hydrated kaolin, but not limited thereto. Preferred kaolinite is a hydrous aluminium silicate and represented as $Al_2O_3 \cdot 2SiO_2 \cdot 2H_2O$. Preferred kaolinite have particle size distribution such that at least 98 wt% of the particles have a particle size of 2 $\mu$m or less. Preferably the kaolinite is a refined kaolin and further preferably the refined kaolin includes 38 wt% $Al_2O_3$ and 45wt% $SiO_2$ and a maximum of 0.5 wt% of $Fe_2O_3$.

Preferably a 10% aqueous solution of the 1:1 layered silicate clay has a pH 5 to 7.

Disclosed toothpaste compositions include 0.1 to 20 wt% more preferably 0.1 to 5 wt% and still more preferably 0.1 to 3 wt% and most preferably 0.1 to 2 wt% of 1:1 layered silicate clay.

2:1 layered silicate clay

Disclosed toothpaste composition includes a 2:1 layered silicate clay. Typically the 2:1 layered silicate clay are swelling clays.

Preferably the 2:1 layered silicate clay is selected from the pyrophyllite-talc group, smectite group, vermiculite group of clay, more preferably the 2:1 layered silicate clay belongs to the smectite group.

The pyrophillite-talc group preferably includes talc which is a hydrous magnesium silicate; pyrophyllite which is a hydrous aluminium silicate; or minnesotaite which is a hydrous iron silicate.

The vermiculite group of clay preferably includes the dioctahedral vermiculite or the trioctahedral vermiculite. The trioctahedral vermiculites preferably has a layer charge of 0.6 to 0.7.

It is highly preferred that the 2:1 layered silicate clay is of the smectite group of clay which includes but not limited to montmorillonite, smectite, beidellite, nontronite saponite, hectorite, sauconite or laponite. The smectite group of clay is preferably dioctahedral smectites or trioctahedral smectites.

Examples of suitable dioctahedral smectites include, but are not limited to, montmorillonite (often referred to as bentonite), smectite, beidellite, nontronite.

Typically montmorillonite and smectite have a low total charge content of from 0.3 to 0.6 and most of the charge originates in the octahedral sheet. Beidellite is an expandable dioctahedral smectite having a total charge content of 0.7 or higher and a tetrahedral charge content of 0.4. When the dioctahedral smectites is a montmorillonite, it is typically carbonate free and preferably water-washed.

Examples of suitable trioctahedral smectites include, but are not limited to, saponite, hectorite, sauconite, and laponite. Trioctahedral smectites having a total charge content of 0.3 to 0.5 includes hectorite which has magnesium and lithium in the octahedral sheet and saponite which has considerable magnesium in the octahedral sheet and some aluminum substitution in the tetrahedral sheet.

A particularly preferred 2:1 layered clay is a smectite group of clay which may be from a natural source, processed or purified from a natural source or prepared synthetically. More preferably the 2:1 layered silicate clay is a magnesium aluminium silicates (various grades are commercially available as VEEGUM® from R. T. Vanderbilt Company); purified sodium magnesium silicates (commercially available as LAPONITE® in various grades); organically modified smectites including tetra alkyl and/or trialkyl ammonium smectites (organically modified montmorillonite clays) such as quaternium-18 bentonite, quaternium-18 hectorite, stearalkonium bentonite and stearalkonium hectorite/ and mixtures thereof. Magnesium aluminium silicates clays are particularly preferred. An example is VEEGUM® HV.

Preferably a 10% aqueous solution of the 2:1 layered silicate clay has a pH of 8 to 10. Disclosed toothpaste compositions include 2:1 layered silicate clay in an amount of 0.1 to 5wt%, more preferably 0.1 to 3wt% and still more preferably 0.1 to 2 wt% and most preferably 0.1 to 1wt%.

Source of phosphate

Preferably the source of phosphate that may be used in this invention is limited only to the extent that the same may be used in a composition suitable for use in an oral cavity. Illustrative examples of the types of phosphate source suitable for use in this invention include monosodium phosphate, sodium dihydrogenphosphate, disodium hydrogenphosphate, sodium pyrophosphate, tetrasodium pyrophosphate, sodium hexametaphosphate, monopotassiunn phosphate, potassium dihydrogenphosphate, dipotassium hydrogenphosphate, trisodium phosphate, tripotassium phosphate, mixtures thereof or the like. The phosphate source is preferably one which is water soluble. Typically, the phosphate source makes up from 0.5 to 15%, and more preferably from 2 to 12%, and most preferably from 4 to 9% by weight of the dentifrice composition, based on total weight of the dentifrice composition and including all ranges subsumed therein. In a most preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios included therein.

Further ingredients

Humectant

Toothpaste in accordance with the invention preferably includes a humectant which is typically a polyol, in particular a polyol selected from glycerol, sorbitol, propylene glycol, xylitol, maltitol, lactitol, or mixtures thereof. Humectants are generally included in toothpastes for a soft and supple mouth feel. Humectants also reduce the tendency of toothpaste composition to lose moisture. Sorbitol is especially preferred, this material also functions as a sweetener, sorbitol is generally available as 70% aqueous solution. The total amount of humectant is typically from 1 to 60 wt%, in particular from 5 to 50 wt%, and especially from 10 wt% to 30 wt% of the total composition.

## Antimicrobial agent

Toothpaste composition in accordance with the invention preferably includes an antimicrobial agent. Examples include triclosan, chlorhexidine, copper, zinc and stannous salts (such as zinc citrate, zinc sulphate, zinc glycinate, sodium zinc citrate, stannous pyrophosphate), sanguinarine extract, metronidazole, quaternary ammonium compounds (such as cetylpyridinium chloride), bis-guanides (such as chlorhexidine), digluconate, hexetidine, octenidine, alexidine; and halogenated bisphenolic compounds (such as 2, 2' -methylenebis- (4-chloro-6-bromophenol)).

## Surfactant

Toothpaste composition in accordance with the invention preferably includes a surfactant.
Suitable surfactants are those which are reasonably stable and provide foam throughout a wider pH range. The surfactant is anionic.
Anionic surfactants useful herein include the water-soluble salts of alkyl sulfates having from 8 to 20 carbon atoms in the alkyl radical (e.g., sodium alkyl sulfate) and the water-soluble salts of sulfonated monoglycerides of fatty acids having from 8 to 20 carbon atoms. Sodium lauryl sulfate and sodium coconut monoglyceride sulfonates are examples of anionic surfactants of this type.
Preferred compositions contain 0.25 to 12 wt%, more preferably from 0.5 to 8 wt%, and most preferably from 1 to about 6 wt% anionic surfactants.
Some anionic surfactants, in particular, sodium lauryl sulphate itself have antibacterial effect. Such action provides some degree of instant antibacterial effect. However, this effect is generally very short-lived. Therefore, the more sustainable antibacterial action is provided by the antibacterial agent such as triclosan.
Other surfactants like nonionic, amphoteric or zwitterionic surfactants may also be included. Nonionic surfactants can be broadly defined as compounds produced by the condensation of alkylene oxide groups (hydrophilic in nature) with an organic hydrophobic compound which may be aliphatic or alkyl- aromatic in nature.
Examples of suitable nonionic surfactants include poloxamers (sold under trade name PLURONIC®), polyoxyethylene, polyoxyethylene sorbitan esters (sold under trade name TWEENS®), POLYOXYL® 40 hydrogenated castor oil, fatty alcohol ethoxylates, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides, and mixtures of such materials. Useful amphoteric surfactants can be broadly described as derivatives of aliphatic secondary and tertiary amines in which the aliphatic radical is a straight chain or branched and wherein one of the aliphatic substituent contains from about 8 to about 18 carbon atoms and one contains an anionic water- solubilizing group, e.g., carboxylate, sulfonate, sulfate, phosphate, or phosphonate. Other suitable amphoteric surfactants are betaines, specifically cocamidopropyl betaine. Mixtures of amphoteric surfactants can also be used.

## Flavouring Agent

Toothpaste composition in accordance with the present invention may advantageously employ a flavouring agent. Flavouring agents which are used in the practice of the present invention include, but are not limited to, essential oils as well as various flavouring aldehydes, esters, alcohols, and similar materials. Examples of the essential oils include oils of spearmint, peppermint, wintergreen, clove, sage, eucalyptus, cinnamon, lemon, lime, and orange. Also useful are such chemicals as menthol, carvone, and anethole. Preferably the flavouring agent is an oil of peppermint, clove, lemon and spearmint.

## Thickening agent

Toothpaste composition in accordance with the present invention may advantageously employ a thickening agent. The disclosed composition preferably contains 0 to 2 wt% thickening silica, which implies that composition may or may not contain thickening silica. Conventional gel toothpaste composition generally contain upto 8.5 wt% thickening silica whereas opaque toothpaste composition typically contain 4 to 10 wt%. The toothpaste compositions can include up to 2 wt% thickening silica. Preferred compositions have 1.5 wt%, or even less than 1 wt% thickening silica. Optimal compositions have less than 0.5 wt% thickening silica. Highly preferred compositions do not contain thickening silica.
When present, preferred thickening silicas include AEROSIL®T series from Degussa or the CAB-O-SIL® series from Cabot Corporation, silica gels such as the SYLODENT® or SYLOX® series from W. R.Grace & Co or precipitated silica such as ZEOTHIX® 265 from J. M. Huber Corporation. Useful silica thickeners also include ZEODENT® 165, ZEODENT® 163 and/or 167 and ZEOFREE® 153, 177, and/or 265 silicas, all available from J. M. Huber Corporation. Other preferred thickening silicas include MFIL®, MFIL®-P (From Madhu Silica), SIDENT® 22 S and AEROSIL® 200 (Ex. Evonik Industries), SYLODENT® and PERKASIL® thickening silicas from WR Grace & Company and Tixosil® 43 and 331 from Rhodia, synthetic finely divided pyrogenic silica such as those sold under the trademarks SYLOID® 244, SYLOID® 266 and AEROSIL® D-200.

Other thickening agents which may also be added to the disclosed composition include one or more other thickening agents such as carboxyvinyl polymers which include carbomers which are commercially available from B. F. Goodrich as the CARBOPOL® series, including CARBOPOL® 934, 940, 941 and 956. Other preferred grades include acrylates/$C_{10-30}$ alkyl acrylate crosspolymers which are commercially available as ULTREZ® 21, PEMULEN® TR-1, and PEMULEN® TR-2, from Noveon Corporation. Preferred compositions can include 0.05 to 10 wt%, more preferably 0.1 to 5 wt%, and even more preferably 0.25 to about 4 wt% of other thickening agents.

### De-sensitising agents

Toothpaste composition in accordance with the present invention may advantageously employ a de-sensitising agent for example potassium salt selected from potassium nitrate, potassium chloride, potassium citrate, potassium tartarate and potassium acetate used preferably from 0.5 to 3 wt%, more preferably from 1 to 2.5 wt % and especially from 1.7 to 2.2 wt%.

### Silicates

Composition in accordance with the invention preferably includes an alkali metal silicate. The alkali metal is sodium or potassium, preferably sodium. Sodium silicate is generally available as 10 to 40 % aqueous solution, most common being 30 % solution. Sodium silicate is available as neutral sodium silicate or alkaline sodium silicate. Preferred toothpastes have neutral sodium silicate. Sodium silicate is available with varying ratios of $Na_2O$: $SiO_2$.

Sodium silicate with $Na_2O$: $SiO_2$ ratio in the range of 3.0 to 3.8 is preferred, more highly preferred range being 3.25 to 3.5. Preferred toothpaste composition include 0.1 to 5 wt% silicate (on dry weight basis). Thus, a 30% solution of sodium silicate is added to the composition in an amount in the range of 0.3 to 16 wt%.

### Binder

Compositions in accordance with the invention preferably include a binder, which lends a good structure to the paste. Cellulosic binders are especially preferred. Preferred cellulosic binders include cellulose ethers, which include hydroxyethyl cellulose (HEC), hydroxypropyl cellulose (HPC), ethyl hydroxyethyl cellulose (EHEC), carboxymethyl cellulose (CMC), carboxymethylhydroxyethyl cellulose (CMHEC), hydroxypropyl hydroxyethyl cellulose (HPHEC), methyl cellulose (MC), methylhydroxypropyl cellulose (MHPC), methylhydroxyethyl cellulose (MHEC), carboxymethyl methyl cellulose (CMMC), hydrophobically modified carboxymethyl cellulose (HMCMC), hydrophobically modified hydroxyethyl cellulose (HMHEC), hydrophobically modified hydroxypropyl cellulose (HMHPC), hydrophobically modified ethyl hydroxyethyl cellulose (HMEHEC), hydrophobically modified carboxymethylhydroxyethyl cellulose (HMCMHEC), hydrophobically modified hydroxypropyl hydroxyethyl cellulose (HMHPHEC), hydrophobically modified methyl cellulose (HMMC), hydrophobically modified methylhydroxypropyl cellulose (HMMHPC), hydrophobically modified methylhydroxyethyl cellulose (HMMHEC), and hydrophobically modified carboxymethylmethyl cellulose (HMCMMC).

Other cellulosic binders include cationic hydroxyethyl cellulose (cationic HEC), cationic hydrophobically modified hydroxyethyl cellulose (cationic HMHEC) and microcrystalline cellulose.

A highly preferred binder is sodium carboxymethyl cellulose (SCMC). Particularly preferred sodium carboxymethyl celluloses include those with degree of substitution of from 0.6 to 0.99, preferably from 0.7 to 0.95.

In addition to, or as a replacement of cellulosic binder, guar gum or its derivatives could be used, however, such binders are less preferred to the cellulosic ones. Such derivatives include carboxymethyl guar (CM guar), hydroxyethyl guar (HE guar), hydroxypropyl guar (HP guar), carboxymethylhydroxypropyl guar (CMHP guar), cationic guar, hydrophobically modified guar (HM guar), hydrophobically modified carboxymethyl guar (HMCM guar), hydrophobically modified hydroxyethyl guar (HMHE guar), hydrophobically modified hydroxypropyl guar (HMHP guar), cationic hydrophobically modified hydroxypropyl guar (cationic HMHP guar), hydrophobically modified carboxymethylhydroxypropyl guar (HMCMHP guar) and hydrophobically modified cationic guar (HM cationic guar).

Other less preferred gums include xanthan gum, carrageenan and derivatives, such as Irish moss and viscarin, gellan gum, sclerotium gum and derivatives, pullulan, rhamsan gum, welan gum, konjac, curdlan, algin, alginic acid, alginates and derivatives, starch phosphate derivatives, agar and derivatives, gum arabic and derivatives, pectin and derivatives, chitosan and derivatives, karaya gum, locust bean gum, natto gum, tragacanth gum, chitin derivatives, gelatin, betaglucan, dextrin, dextran, cyclodextrin and polyquaterniums, furcellaren gum, ghatti gum, psyllium gum, quince gum, tamarind gum, larch gum, and tara gum.

Preservatives

Toothpaste composition with calcium containing abrasives especially chalk are prone to bacterial growth. Certain preservatives, e.g. methyl, ethyl, butyl, propyl and isopropyl esters of parahydroxybenzoic acid may be particularly useful against bacterial growth. A mixture of methyl, ethyl, butyl and propyl esters of parahydroxybenzoic acid is particularly preferred. The activity of this mixture can be enhanced by adding phenoxyethanol. Formaldehyde and dimethyl hydantoin are other preferred preservatives. Preservatives are generally included at a level of 0.05 to 0.8 wt% in the composition.

Sweetening agent

Compositions in accordance with the present invention may also contain a sweetening agent. Preferred sweetening agents include sodium saccharin, aspartame, sucralose, thaumatin, acesulfame potassium, stevioside, stevia extract, paramethoxy cinnamic aldehyde, neohesperidyl dihydrochalcone and perillartine. Typical levels are from 0.005 to 5 wt%, more preferably from 0.01 to 1 wt%.

Toothpaste composition

Disclosed toothpaste composition may be transparent, opaque or translucent depending on the type of abrasive system used.

A toothpaste composition in accordance with the present invention preferably includes a ratio by weight of the 1:1 layered silicate clay to the 2:1 layered silicate clay in the range of 1.5 to 5, more preferably from 1.5 to 3, still more preferably from 1.6 to 3, still more preferably from 1.6 to 2 and most preferably from 1.8 to 2.

A toothpaste composition in accordance with the present invention preferably includes a ratio by weight of the 1:1 layered silicate clay to the source of fluoride in the range of 1.5 to 15, more preferably from 1.5 to 5, still more preferably from 1.6 to 5, still more preferably from 1.6 to 2 and most preferably from 1.8 to 2.

A toothpaste composition in accordance with the present invention preferably includes a ratio by weight of the 2:1 layered silicate clay to the source of fluoride in the range of 1 to 10, more preferably from 1 to 6, still more preferably from 1.05 to 5, still more preferably from 1.05 to 4 and most preferably from 1.05 to 2.

Method of using the toothpaste composition

According to a second aspect of the present invention, disclosed is a method of promoting remineralisation of teeth including the steps of: (i) contacting teeth with the toothpaste composition according to the first aspect; and, (ii) optionally, rinsing the oral cavity with a suitable solvent. Preferably the solvent is water.

The method may be therapeutic in nature. Alternatively, and more preferably, the method is non-therapeutic or cosmetic in nature.

According to a third aspect disclosed is a use of a 1:1 layered silicate clay together with a 2:1 layered silicate clay in a toothpaste composition having an abrasive silica or a calcium based abrasive and a source of fluoride for promoting remineralization of teeth.

It is preferred that the use is for non-therapeutic or cosmetic purpose.

According to a fourth aspect disclosed is a use of a 1:1 layered silicate clay together with a 2:1 layered silicate clay in a toothpaste composition having an abrasive silica or a calcium based abrasive and a source of fluoride for decreasing demineralization of teeth.

It is preferred that this use is for non-therapeutic or cosmetic purpose.

The invention will be further illustrated in the following non-limiting examples.

**Examples**

Four different toothpaste compositions with ingredients as indicated in Table 1 were prepared. Of the four toothpaste compositions; Composition A, B and C are comparative compositions and the Composition 1 is according to the present invention. Each of the toothpaste composition as provided in Table 1 were used in the demineralisation and remineralisation study as is provided below.

a) Demineralisation study

Labial surfaces of sound bovine incisors were ground flat and polished with alumina slurry. Four sections were cut from each tooth, and test regions were created on the polished surfaces by painting the rest of the tooth section with acid-resistant nail varnish to provide the teeth sample for the study. Micro-hardness of the prepared teeth sample were analysed and recorded. The teeth sample were subsequently washed with copious amounts of Milli-Q water and thereafter subjected to a pH-cycling regime as follows:

- First the teeth sample was immersed for 5 minutes in toothpaste slurry. The toothpaste slurry was prepared by mixing 1 part of toothpaste composition with 3 parts of deionised water having 10 UI/mL alkaline phosphatase.
- Next the teeth sample was immersed for 1 hour in an acidic buffer. The acidic buffer was prepared by mixing 50 mmol/L acetic acid and 1.5 mmol/L $KH_2PO_4$; pH5.0.
- After this, the teeth sample was immersed for 1 minute in a neutral buffer. The neutral buffer was prepared by mixing 20 mmol/L HEPES and 1.5 mmol/L $KH_2PO_4$; pH 7.0.
- This cycle was repeated 12 times.
- Teeth sample was washed after each step to thoroughly rinse off the active agents from the previous steps.
- Efficacy was assessed by micro-hardness analysis of the teeth sample, before and after pH-cycling.
- 4 readings were taken for each teeth sample and demineralisation expressed as a percentage reduction in hardness (% HK Reduction).
- The results of the %HK reduction for each toothpaste composition is provided in table 1.

b) Remineralisation study

Labial surfaces of sound bovine incisors were ground flat and polished with alumina slurry. Four sections were cut from each tooth, and regions were created on the polished surfaces by painting the rest of the tooth section with acid-resistant nail varnish to provide the teeth sample for the study.

The teeth sample was demineralised for 10 days in a gel system prepared from equal parts of 8% methyl cellulose and lactic acid, at 37°C and a pH 4.6. After demineralisation each teeth sample was indented in order to obtain the Hardness Knoop value of the teeth sample.

After lesion preparation, teeth sample were subjected to a pH-cycling regime as follows:

- First the teeth sample was immersed for 5 minutes in toothpaste slurry. The toothpaste slurry was prepared by mixing 1 part of toothpaste composition with 3 parts of deionised water having 10 UI/mL alkaline phosphatase.
- Next the teeth sample was immersed for 30 minutes in an acidic buffer. The acidic buffer was prepared by mixing 50 mmol/L acetic acid, 1.5 mmol/L calcium chloride dihydrate, 0.9 mmol/L potassium dihydrogen orthophosphate and 130 mmol/L potassium chloride, pH 5.0)
- Thereafter the teeth sample was immersed for 10 minutes in a neutral buffer. The neutral buffer was prepared by mixing 20 mmol/L HEPES, 1.5 mmol/L calcium chloride dihydrate, 0.9 mmol/L potassium dihydrogen orthophosphate and 130 mmol/L potassium chloride, pH 7.0).
- Efficacy was assessed by micro-hardness analysis of the teeth sample, before and after the pH-cycling regime as described above.
- 4 readings were taken for each teeth sample and the remineralisation was expressed as a % of hardness Knoop (Hardness Knoop = HK) restored, as follow:

$$\% \text{ HK Restored} = (\Delta \text{ HK } / \text{ HK Baseline}) * 100$$

where $\Delta$ HK was calculated as follow: HK after - HK before treatment.

**TABLE 1**

| Composition | A (wt%) | B (wt%) | C (wt%) | 1 (wt%) |
|---|---|---|---|---|
| FGNC | 40.00 | 40.00 | 40.00 | 40.00 |
| SMFP | 0.76 | 0.76 | 0.76 | 0.76 |
| Smectite clay | 0 | 0.80 | 0 | 0.80 |
| Kaolin clay | 0 | 0 | 1.5 | 1.5 |
| Other ingredients | Upto 100.00 | Upto 100.00 | Upto 100.00 | Upto 100.00 |
| %HK restored (Remineralisation) | 36.1 | 38.01 | 37.16 | 42.01 |

(continued)

| Composition | A (wt%) | B (wt%) | C (wt%) | 1 (wt%) |
|---|---|---|---|---|
| %HK reduction (Demineralisation) | 31.45 | 30.62 | 23.33 | 23.44 |

The results in Table 1 clearly shows that composition 1 which is according to the present invention having both a 1:1 layered silicate clay and a 1:1 layered silicate clay provides improved remineralisation of tooth sample as compared to the comparative composition (A, B, C). The composition 1 according to the present invention also reduces the levels of demineralisation of tooth sample.

**Claims**

1. A toothpaste composition comprising:

   (i) 2 to 70 wt% abrasive;
   (ii) a fluoride source;
   (iii) 1:1 layered silicate clay; and,
   (iv) 2:1 layered silicate clay.
   wherein the abrasive is an abrasive silica or a calcium based abrasive.

2. A composition as claimed in claim 1 wherein the 1:1 layered silicate is a kaolinites clay.

3. A composition as claim 2 wherein the kaolinites clay is selected from kaolinite, halloysite, nacrite and dickite.

4. A composition as claimed in claim 3 wherein the kaolinite is a hydrated aluminium silicate.

5. A composition as claimed in claim 1 wherein the 2:1 layered silicate clay is from the smectite group of clay.

6. A composition as claimed in claim 5 wherein the clay from the smectite group of clay is montmorillonite, smectite, beidellite, nontronite, saponite, hectorite, sauconite, or laponite.

7. A composition as claimed in claim 5 or 6 wherein the clay from smectite group of clay is magnesium aluminium silicate.

8. A composition as claimed in any preceding claim 1 to 7 wherein the 1:1 layered silicate clay is present in an amount of 0.1 to 20 % by weight of the composition.

9. A composition as claimed in any preceding claim 1 to 8 wherein the 2:1 layered silicate clay is present in an amount of 0.1 to 5% by weight of the composition.

10. A composition as claimed in any preceding claim 1 to 9 wherein the source of fluoride is selected from sodium monofluorophosphate, sodium flouride or stannous fluoride.

11. A composition as claimed in any preceding claim 1 to 10 wherein said composition comprises a calcium source.

12. A 1:1 layered silicate clay together with a 2:1 layered silicate clay in a toothpaste composition comprising an abrasive silica or a calcium based abrasive and a fluoride source for use in promoting remineralization of teeth.

13. A 1:1 layered silicate clay together with a 2:1 layered silicate clay in a toothpaste composition comprising an abrasive silica or a calcium based abrasive and a fluoride source for use in decreasing demineralization of tooth.

**Patentansprüche**

1. Zahnpastazusammensetzung, umfassend:

   (i) 2 bis 70 Gew.-% Schleifmittel;

(ii) eine Fluorid-Quelle;
(iii) einen 1:1-Schichtsilikat-Ton; und
(iv) einen 2:1-Schichtsilikat-Ton,
wobei das Schleifmittel ein abrasives Siliziumdioxid oder ein auf Calcium basierendes Schleifmittel ist.

2. Zusammensetzung, wie im Anspruch 1 beansprucht, wobei das 1:1-Schichtsilikat ein Kaolinit-Ton ist.

3. Zusammensetzung, wie im Anspruch 2 beansprucht, wobei der Kaolinit-Ton unter Kaolinit, Halloysit, Nakrit und Dickit ausgewählt ist.

4. Zusammensetzung, wie im Anspruch 3 beansprucht, wobei das Kaolinit ein hydratisiertes Aluminiumsilikat ist.

5. Zusammensetzung, wie im Anspruch beansprucht, wobei der 2:1-Schichtsilikat-Ton aus der Smektit-Gruppe von Ton stammt.

6. Zusammensetzung, wie im Anspruch 5 beansprucht, wobei der Ton aus der Smektit-Gruppe von Ton Montmorillonit, Smektit, Beidellit, Nontronit, Saponit, Hektorit, Sauconit oder Laponit ist.

7. Zusammensetzung, wie im Anspruch 5 oder 6 beansprucht, wobei der Ton aus der Smektit-Gruppe von Ton Magnesiumaluminiumsilicat ist.

8. Zusammensetzung, wie in irgendeinem vorhergehenden Anspruch 1 bis 7 beansprucht, wobei der 1:1-Schichtsilicat-Ton in einer Menge von 0,1 bis 20 Gewichts-% der Zusammensetzung vorliegt.

9. Zusammensetzung, wie in irgendeinem vorhergehenden Anspruch 1 bis 8 beansprucht, wobei der 2:1-Schichtsilicat-Ton in einer Menge von 0,1 bis 5 Gewichts-% der Zusammensetzung vorliegt.

10. Zusammensetzung, wie in irgendeinem vorhergehenden Anspruch 1 bis 9 beansprucht, wobei die Fluorid-Quelle unter Natriummonofluorphosphat, Natriumfluorid oder Zinnfluorid ausgewählt ist.

11. Zusammensetzung, wie in irgendeinem vorhergehenden Anspruch 1 bis 10 beansprucht, wobei die Zusammensetzung eine Calcium-Quelle umfasst.

12. 1:1-Schichtsilicat-Ton zusammen mit einem 2:1-Schichtsilicat-Ton in einer Zahnpastazusammensetzung, umfassend ein abrasives Siliciumdioxid oder ein auf Calcium basierendes Schleifmittel und eine Fluorid-Quelle zur Verwendung bei der Förderung der Remineralisierung von Zähnen.

13. 1:1-Schichtsilicat-Ton zusammen mit einem 2:1-Schichtsilicat-Ton in einer Zahnpastazusammensetzung, umfassend ein abrasives Siliciumdioxid oder ein auf Calcium basierendes Schleifmittel und eine Fluorid-Quelle zur Verwendung bei dem Verringern der Demineralisierung von Zähnen.

**Revendications**

1. Composition de dentifrice comprenant :

(i) de 2 à 70 % en masse d'abrasif ;
(ii) une source de fluorure ;
(iii) de l'argile de silicate stratifié 1:1 ; et,
(iv) de l'argile de silicate stratifié 2:1
dans laquelle l'abrasif est une silice abrasive ou un abrasif à base de calcium.

2. Composition selon la revendication 1, dans laquelle le silicate stratifié 1:1 est une argile de kaolinites.

3. Composition selon la revendication 2, dans laquelle l'argile de kaolinites est choisie parmi la kaolinite, l'halloysite, la nacrite et la dickite.

4. Composition selon la revendication 3, dans laquelle la kaolinite est du silicate d'aluminium hydraté.

**5.** Composition selon la revendication 1, dans laquelle l'argile de silicate stratifié 2:1 est de l'argile du groupe des smectites.

**6.** Composition selon la revendication 5, dans laquelle l'argile de l'argile du groupe des smectites est la montmorillonite, la smectite, la beidellite, la nontronite, la saponite, l'hectorite, la sauconite, ou la laponite.

**7.** Composition selon la revendication 5 ou 6, dans laquelle l'argile de l'argile du groupe des smectites est du silicate de magnésium aluminium.

**8.** Composition selon l'une quelconque des revendications 1 à 7, dans laquelle l'argile de silicate stratifié 1:1 est présente dans une quantité de 0,1 à 20 % en masse de la composition.

**9.** Composition selon l'une quelconque des revendications 1 à 8 précédentes, dans laquelle l'argile de silicate stratifié 2:1 est présente dans une quantité de 0,1 à 5 % en masse de la composition.

**10.** Composition selon l'une quelconque des revendications 1 à 9 précédentes, dans laquelle la source de fluorure est choisie parmi le monofluorophosphate de sodium, le fluorure de sodium ou le fluorure stanneux.

**11.** Composition selon l'une quelconque des revendications 1 à 10 précédentes, dans laquelle ladite composition comprend une source de calcium.

**12.** Argile de silicate stratifié 1:1 avec une argile de silicate stratifié 2:1 dans une composition de dentifrice comprenant une silice arbrasive ou un abrasif à base de calcium et une source de fluorure pour une utilisation dans la promotion de reminéralisation des dents.

**13.** Argile de silicate stratifié 1:1 avec une argile de silicate stratifié 2:1 dans une composition de dentifrice comprenant une silice abrasive ou un abrasif à base de calcium et une source de fluorure pour une utilisation dans la diminution de déminéralisation des dents.

**EP 3 393 426 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4455293 B, Harvey
- EP 0102695 A2
- US 4064231 A
- US 4122163 A